# EUROPÄISCHE PATENTANMELDUNG

(11) **EP 0 526 919 A1**
(43) Veröffentlichungstag der Anmeldung: **10.02.1993**
(21) Anmeldenummer: 92201078.0
(22) Anmeldetag: 15.04.1992
(51) Int. Cl.: A61F 9/06

(54) **Schweissschutz-Arbeitsschutz-Helm aus Kunststoff in beliebiger Farbe mit klarer oder getönter Sichtscheibe**

(30) Priorität: 07.08.1991 DE 9109795 U
(71) Anmelder: PROFIL - Jean-Paul Burlet Gmbh, D-63755 Alzenau (DE)
(72) Erfinder: Burlet, Jean-Paul, W-8755 Alzenau (DE)
(74) Vertreter: Falge, Dieter, Dr.

(57) **Zusammenfassung**

Schweißschutz/Arbeitsschutz-Helme mit Sichtscheibe sind derzeit so gestaltet, daß bei Beschädigung der Sichtscheibe durch Schweiß-Funken, mechanische Beschädigungen oder sonstige Einflüsse die Sichtscheibe von der Innenseite des Helms her mit erheblichem Arbeits- und Zeitaufwand ausgewechselt werden muß. Sofern beim Schweißschutz- Helm zusätzlich eine mit Fotozellen versehene Kassette in den Helm eingebaut ist, muß diese beim Wechseln der Klarsichtscheibe von innenher ebenfalls ausgebaut werden.

Der hier zur Anmeldung gebrachte Schweißschutz-/Arbeitsschutz-Helm ist so konstruiert, daß die elastische Sichtscheibe von außen (vorne) in Führungsschienen mit einer leichten Wölbung der Klarsichtscheibe nach außen eingelegt wird.

Die leichte Wölbung der Klarsichtscheibe gewährt dieser eine Spannung, wodurch der Andruck an die Führungen und Halteschienen und damit auch eine Staubdichte gewährleistet ist, ohne daS es durch die leichte Wölbung der Sichtscheibe zu Bildverzerrungen kommt. Das Ausbauen einer mit Fotozellen versehenen Kassette im Schweißschutz-Helm erübrigt sich durch die Möglichkeit des Auswechselns der Sichtscheibe von außen.

## Beschreibung

Die Erfindung bezieht sich auf einen Schutzhelm aus Kunststoff, der bei Schweißarbeiten oder Arbeiten, bei denen Staub oder Splitter gefährden, als Gesichtsschutz eingesetzt wird.

Der derzeitige Stand der Technik ist der, daß die bisher zum Einsatz gekommenen SchweißSchutz-/Arbeitsschutzhelme mit einer glatten, ebenen Frontseite, in welche das aus klarem oder durchsichtigem Kunststoff bestehende Sichtfenster eingelassen ist, versehen sind.

Die Kunststoffsichtscheiben werden durch Schweißfunkenpartikel oder sonstige mechanische Vorgänge beschädigt und müssen je nach Grad der Beschädigung mehr oder weniger häufig ausgewechselt werden, was dadurch geschieht, daß die Befestigungen im Innenteil des Helms gelöst, die defekte Klarsichtscheibe entfernt und durch eine neue Klarsichtscheibe, die von innen wieder eingesetzt und befestigt wird, ersetzt wird.

Die Kritik an der vorstehend beschriebenen Gestaltung und Verfahrensweise beim Wechseln der Klarsichtscheibe richtet sich darauf, daß der mit dem von der Innenseite des Helms durchzuführenden Austausch der Klarsichtscheibe eine erheblicher Arbeits- und Zeitaufwand verbunden ist. Beim Schweißschutzhelm kommt hinzu, daß zum Schutz der Augen des Arbeiters eine mit Fotozellen versehene Kassette in den Helm eingebaut ist, die beim Wechseln der Klarsichtscheibe von innen ebenfalls ausgebaut werden muß.

Die Aufgabe der Erfindung besteht darin, die vorstehend genannten Nachteile zu beseitigen und die Möglichkeit zu schaffen, ein Auswechseln der Klarsichtscheibe innerhalb kürzester Zeit zu gewährleisten, ohne Befestigungsschrauben oder Klammern lösen und ohne beim Schweißschutz- helm eingebaute fotomechanische Kassetten zuvor ausbauen zu müssen.

Die Lösung dieses Ziels wird dadurch erreicht, daß die elastische Sichtscheibe von außen (vorne) in Führungsschienen mit einer leichten Wölbung der Klarsichtscheibe nach außen eingelegt wird. Die leichte Wölbung gewährt eine Spannung der Klarsichtscheibe, wodurch der Andruck der Klarsichtscheibe an die Führungen und Haltschienen und damit eine Staubdichte gewährleistet ist, ohne daß es durch die leichte Wölbung der Sichtscheibe zu Bildverzerrungen kommt.

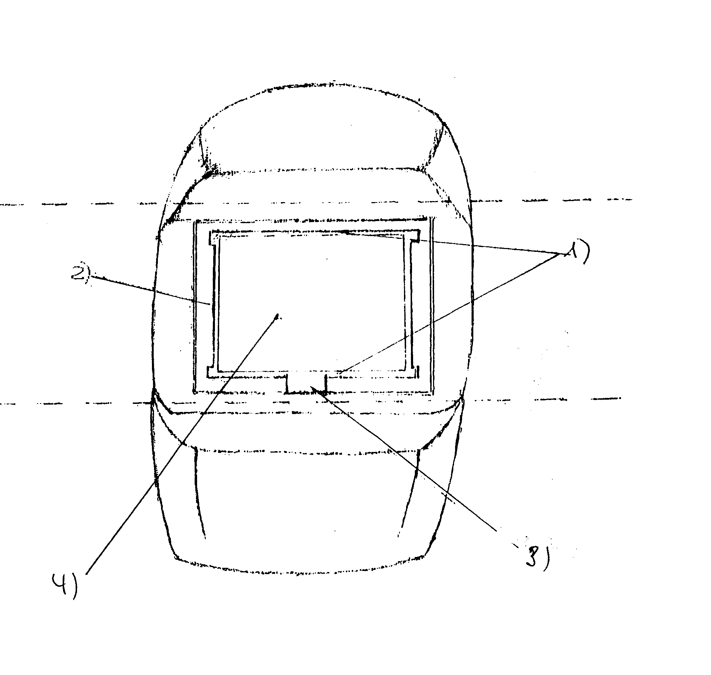

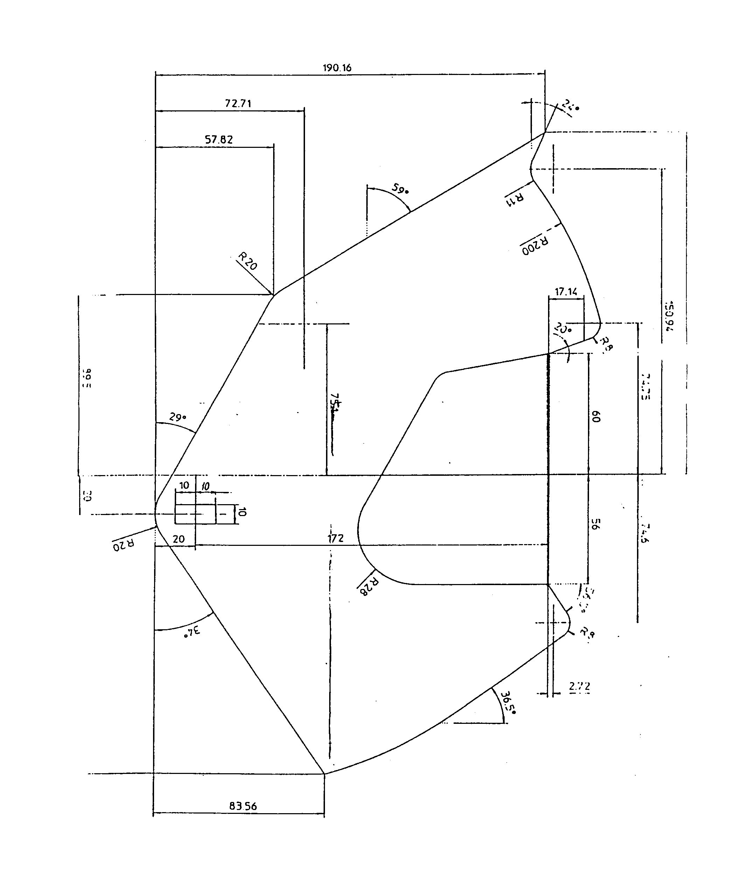

Die beigefügten Zeichnungen geben den Helm in Frontansicht (1) sowie in Seitenansicht (2) wieder. In der Zeichnung (3) ist die konstruktive Lösung für die Halterung der einzulegenden Klarsichtscheibe dargestellt.

### Bezugszeichenliste:

zum Schweißschutz-Arbeitsschutzhelm
1 = nach außen leicht gewölbte Radien
2 = Hinterschnitte zur Aufnahme der Schichtscheibe
3 = Griffmulde zum Entfernen der Sichtscheibe
4 = elastische Sichtscheibe

## Patentansprüche

1. Schweißschutz-/Arbeitsschutz-Helm aus Kunststoff in beliebiger Farbe mit klarer oder getönter Sichtscheibe, dadurch gekennzeichnet,
daß die elastische Sichtscheibe des Helmes von außen (vorne) in die dafür vorgesehenen Halteschienen eingelegt bzw. aus diesen beim Auswechseln wieder herausgenommen werden kann.
